# EUROPÄISCHE PATENTANMELDUNG

(11) **EP 0 784 203 A2**
(43) Veröffentlichungstag der Anmeldung: **16.07.1997**
(21) Anmeldenummer: 96119266.3
(22) Anmeldetag: 02.12.1996
(51) Int. Cl.: G01N 33/00

(54) **Einrichtung zum Angleichen von Messergebnissen**

(30) Priorität: 10.01.1996 DE 19600648
(71) Anmelder: ROBERT BOSCH GMBH, 70442 Stuttgart (DE)
(72) Erfinder: Kleger, Bruno, 73340 Amstetten (DE); Mai, Hans, 73035 Goeppingen (DE); Nobis, Guenter, 73240 Wendlingen (DE); Maier, Michael, 73240 Wendlingen (DE); Peiler, Juergen, 72644 Oberbohingen (DE)

(57) **Zusammenfassung**

Es wird eine Einrichtung zum Angleichen von Meßergebnissen zwischen einem ersten, als Referenzgerät dienenden Gasanalysator und einem zweiten Gasanalysator (10) mit einer Meßanordnung vorgeschlagen, deren ermittelten Untersuchungsergebnisse in einer signalverarbeitenden Anordnung auswertbar sind, mit einer Gaseinlaßöffnung (13) und einer Gasauslaßöffnung (15). Dabei ist ein eine Gaseinlaßöffnung (14) und zwei Gasauslaßöffnungen (18, 20) aufweisender Hohlkörper (21) vorgesehen, dessen erste Gasauslaßöffnung (18) mit der Gaseinlaßöffnung (13) des zweiten Gasanalysators (10) kommuniziert, während die zweite Gasauslaßöffnung (20) ins Freie (24) bzw. in die Gasauslaßöffnung (15) des Gasanalysators (10) führt und eine definierte Größe aufweist.

## Beschreibung

### Stand der Technik

Die Erfindung geht aus von einer Einrichtung zum Angleichen von Meßergebnissen zwischen zwei Gasanalysatoren nach der Gattung des Hauptanspruches. Ferner betrifft die Erfindung einen Gasanalysator mit einer solchen Einrichtung.

Es ist bekannt, daß zur Messung von Autoabgasen als Referenzgeräte dienende Gasanalysatoren, also Vorrichtungen zur Messung der Trübung von Rauchgas, eingesetzt werden, die jedoch schon aufgrund ihrer geringen Stückzahl und vor allem wegen der Entfernung von der überwiegenden Mehrzahl der Benutzer nicht ohne weiteres in Anspruch genommen werden können. Andererseits sind Gasanalysatoren bekannt und zum Beispiel in der DE 43 21 717 A 1 beschrieben, die zur Untersuchung von Gasen, insbesondere von Diesel-Motoren angetriebenen Kraftfahrzeugen, einsetzbar sind, jedoch wegen ermittelter Untersuchungsergebnisse, die vom Referenzgerät abweichen können, nicht ohne weiteres verwendbar sind.

Der Erfindung liegt die Aufgabe zugrunde, eine Einrichtung zum Angleichen von Meßergebnissen zwischen zwei Gasanalysatoren anzugeben, bei der die Angleichung mit minimalem technischen Aufwand durchgeführt werden kann. Eine weitere Aufgabe der Erfindung besteht darin, einen Gasanalysator vorzuschlagen, dessen Meßergebnisse den Messergebnissen eines Referenzgeräts durch Anpassung der physikalischen Ansprechzeit problemlos angeglichen werden können.

Die Aufgabe wird erfindungsgemäß durch die in unabhängigen Ansprüchen angegebenen Merkmale gelöst.

### Vorteile der Erfindung

Mit der vorgeschlagenen Einrichtung (Bypaß), die vor der eingentlichen Meßkammer angebracht ist, wird der Volumenstrom des zu analysierenden Gases durch die Messkammer variiert und das physikalische Ansprechverhalten herkömmlicher Gasanalysatoren an das bei der amtlichen Zertifizierung zu Vergleichen herangezogene Referenzgerät angepaßt. Die erfindungsgemäße Einrichtung sieht hierzu einen eine Gaseinlaßöffnung und zwei Gasauslaßöffnungen aufweisenden Hohlkörper vor, dessen erste Gasauslaßöffnung mit der Gaseinlaßöffnung des Gasanalysators kommuniziert, während die zweite Gasauslaßöffnung ins Freie bzw. in die Auslaßöffnung des Gasanalysators führt und eine definierte Größe aufweist. Da bei montierter Einrichtung (Bypaß) nur ein Teil des Abgases in die Meßkammer geleitet wird, kann die Verschmutzung einzelner Teile des Gasanalysators, insbesondere des Ventils, des Meßrohres und der Optik, minimiert werden. Dies führt zu einer Erhöhung der Standzeit.

Die Reinigungsintervalle des Gasanalysators werden verlängert. Durch die Einrichtung wird der Gegendruck im Auspuff des Kraftfahrzeuges reduziert, die Folge davon ist eine höhere Strömungsgeschwindigkeit im Entnahmeschlauch, wodurch die Abkühlung des Abgases im Schlauch geringer ausfällt. Hinzu kommt noch, daß der Schlauch permanent, daß heißt auch im Leerlauf, durchströmt wird. Dies hat zur Folge, daß eine Abkühlung von Schlauch und Abgas vermindert wird. Die Einrichtung (Bypaß) reduziert den Durchfluß durch die Meßkammer, was zur Abnahme der Strömungsgeschwindigkeit führt. Die physikalische Ansprechzeit wird verlängert.

Man erkennt, daß die Erfindung jedenfalls dann verwirklicht ist, wenn die Einrichtung zum Beispiel an der Vorderseite des Gasanalysators vor dem eigentlichen Abgas-Eingangstutzen angeordnet ist und mit dem Gasanalysator zum Beispiel verschraubt wird. Dadurch strömt nur ein Teil des aus dem Auspuff entnommenen Abgases in die Meßkammer hinein. Das restliche Abgas wird ins Freie bzw. in die Auslaßöffnung des Gasanalysators abgezweigt, wo es bei Bedarf abgesaugt werden kann. Um den Einfluß einer solchen Absaugvorrichtung gering zu halten, ist die Verbindung zwischen Bypaß-Stutzen und Absaugrohr undicht, was zum Beispiel dadurch erreicht wird, daß die Bohrungsdurchmesser unterschiedlich sind und der Spalt zwischen den beiden Bohrungen wenige mm, z.B. 3 mm, beträgt. Die Gaseinlaßöffnung der Erfindung und die Gaseinlaßöffnung des Gasanalysators sind gegeneinander versetzt.

Vorteilhafte Weiterbildungen und Verbesserungen der erfindungsgemäßen Einrichtung ergeben sich aus Unteransprüchen.

Dadurch, daß der Hohlkörper einen Gas-Verteilungsraum definiert und einen Anschlußflansch zur betriebsgemäßen Verbindung mit dem Gasanalysator aufweist, wird zum einen das Abgas gezielt verteilt und zum anderen ist das Anbringen der Einrichtung beziehungsweise deren Demontage einfach durchführbar. Hierbei können diese Maßnahmen auch so getroffen sein, daß der Anschlußflansch die erste Gasauslaßöffnung des Hohlkörpers umgrenzt, in die der Gaseinlaß des Gasanalysators hineinragt.

Weitere vorteilhafte Weiterbildungen sowie Ausgestaltung der erfindungsgemäßen Einrichtung ergeben sich aus weiteren Unteransprüchen in Verbindung mit der folgenden Beschreibung.

### Zeichnung

Die Erfindung ist anhand des in der Zeichnung dargestellten Ausführungsbeispiels in der nachfolgenden Beschreibung näher erläutert. Es zeigen in schematischer Darstellung
- Fig. 1: die Vorderansicht eines Gasanalysators mit einer Einrichtung,
- Fig. 2: einen Schnitt entlang der Line II - II nach Fig. 1 und
- Fig. 3: die Unterseite der in Fig. 1 dargestellten Einrichtung.

### Beschreibung des Ausführungsbeispiels

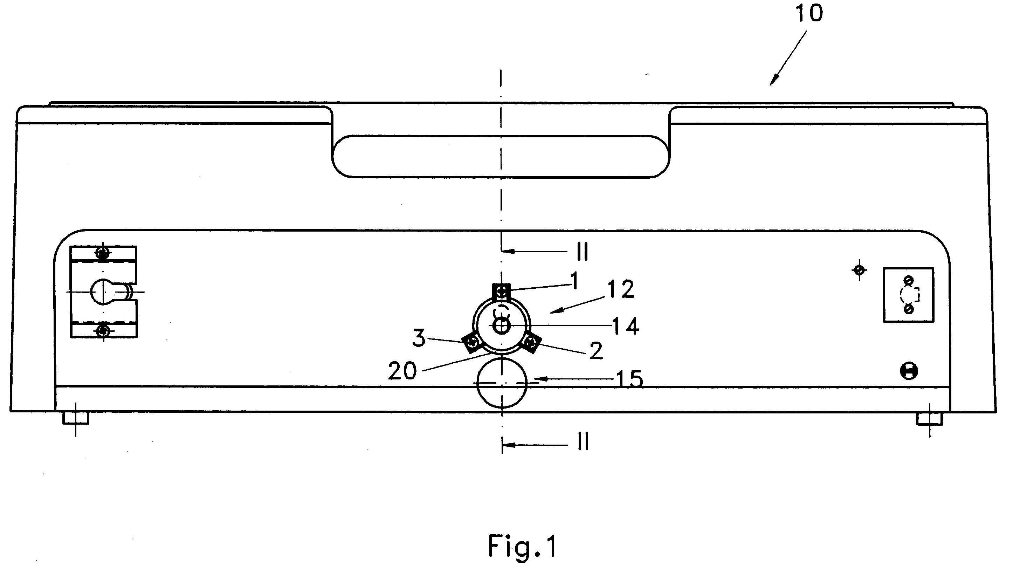

In Figur 1 ist ein herkömmlicher Gasanalysator mit einer nicht näher dargestellten Meßanordnung gezeigt, durch die das zu untersuchende Gas geleitet wird.

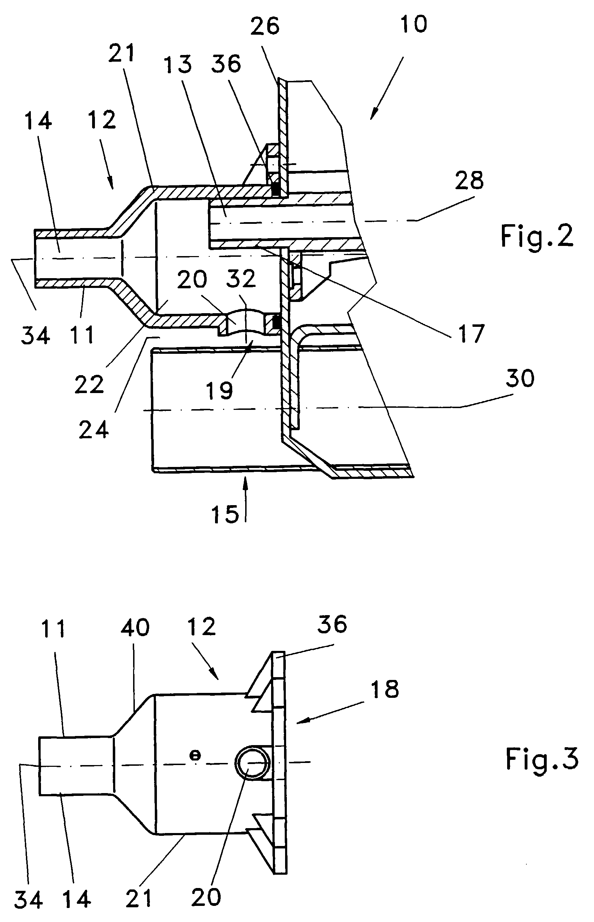

Das von der Meßanordnung ermittelte Untersuchungsergebnis wird in einer signalverarbeitenden Anordnung in Abhängigkeit von anderen Meßergebnissen korrigiert. Die Einrichtung 12, wie sie in den Figuren 1 bis 3 dargestellt ist, dient zum Angleichen von Meßergebnissen zwischen zwei Gasanalysatoren. Der erste, nicht näher dargestellte und als Referenzgerät dienende Gasanalysator mißt dieselben Abgase wie der zweite Gasanalysator 10, der eine Gaseinlaßöffnung 13 und eine Gasauslaßöffnung 15 aufweist. Die Einrichtung besitzt einen, eine Gaseinlaßöffnung 14 und zwei Gasauslaßöffnungen 18, 20, aufweisenden Hohlkörper 21, dessen erste Gasauslaßöffnung 18 mit der Gaseinlaßöffnung 13 des zweiten Gasanalysators 10 kommuniziert, während die zweite Gasauslaßöffnung 20 ins Freie 24 bzw. in die Gasauslaßöffnung 15 des Gasanalysators 10 führt und eine definierte Größe aufweist. Der Hohlkörper 21 definiert einen Gas-Verteilerraum 22 und weist einen Anschlußflansch 36 zur betriebsgemäßen Verbindung mit dem zweiten Gasanalysator 10. Dabei ist der Anschlußflansch 36 so bemessen, daß er die erste Gasauslaßöffnung 18 umgrenzt, in welche die Gaseinlaßöffnung 13 des Gasanalysators 10 hineinragt. Man erkennt, daß gegenüber der Gaseinlaßöffnung 13 die Gaseinlaßöffnung 14 der Einrichtung 12 versetzt angeordnet ist, die durch einen Stutzen 11 definiert ist. Ferner läßt Figur 2 erkennen, daß die Gaseinlaßöffnung 13 des zweiten Gasanalysators 10 durch einen in den Gas-Verteilerraum 22 hineinragenden Stutzen 17 definiert ist, so daß die zweite Gasauslaßöffnung 20 in bezug auf die Gaseinlaßöffnung 13 des Gasanalysators 10 seitlich angeordnet ist.

Die zweite Gasauslaßöffnung 20 der Einrichtung 12 kommuniziert mit einer in der Gasauslaßleitung 15 ausgebildeten Öffnung 19 des Gasanalysators 10. Dabei sind die beiden Öffnungen 19 und 20 annähernd koaxial zueinander angeordnet.

Aus den Figuren geht auch hervor, daß die Einrichtung 12 ein rotationssymmetrischer Körper ist, bei dem die Einlaßöffnung 14 axial zur Achse 34 angeordnet ist, während der Einlaßstutzen 17 der Gaseinlaßöffnung 13 des Gasanalysators 10 parallel zur Rotationsachse 34 angeordnet ist. Im übrigen sind die Achsen 28, 30 und 34 parallel zueinander angeordnet. Die zweite Gasauslaßöffnung 20 ist im zylindrischen Mantel ausgebildet, wobei ihre Achse in etwa rechtwinkelig zur Rotationsachse 34 verläuft. Die Einrichtung 12 ist durch ihren Anschlußflansch 36 an der Wand 26 mittels Schrauben 1, 2, 3 lösbar befestigt.

## Patentansprüche

1. Einrichtung (12) zum Angleichen von Meßergebnissen zwischen einem ersten, als Referenzgerät dienenden Gasanalysator und einem zweiten Gasanalysator (10) mit einer Meßanordnung, deren ermittelten Untersuchungsergebnisse in einer signalverarbeitenden Anordnung auswertbar sind, mit einer Gaseinlaßöffnung (13) und einer Gasauslaßöffnung (15),
gekennzeichnet durch
einen eine Gaseinlaßöffnung (14) und zwei Gasauslaßöffnungen (18, 20) aufweisenden Hohlkörper (21), dessen erste Gasauslaßöffnung (18) mit der Gaseinlaßöffnung (13) des zweiten Gasanalysators (10) kommuniziert, während die zweite Gasauslaßöffnung (20) ins Freie (24) bzw. in die Gasauslaßöffnung (15) des Gasanalysators (10) führt und eine definierte Größe aufweist.

2. Einrichtung nach Anspruch 1,
dadurch gekennzeichnet,
daß der Hohlkörper (21) einen Gas-Verteilerraum (22) definiert und einen Anschlußflansch (36) zur betriebsgemäßen Verbindung mit dem zweiten Gasanalysator (10) aufweist.

3. Einrichtung nach Anspruch 1 oder 2,
dadurch gekennzeichnet,
daß der Anschlußflansch (36) die erste Gasauslaßöffnung (18) umgrenzt, in die die Gaseinlaßöffnung (13) des Gasanalysators (10) hineinragt.

4. Einrichtung nach einem der Ansprüche 1 bis 3,
dadurch gekennzeichnet,
daß gegenüber der Gaseinlaßöffnung (13) die Gaseinlaßöffnung (14) der Einrichtung (12) versetzt angeordnet ist, die durch einen Stutzen (11) definiert ist.

5. Einrichtung nach einem der Ansprüche 1 bis 4, wobei die Gaseinlaßöffnung (13) des zweiten Gasanalysators (10) durch einen in den Gas-Verteilerraum (22) hineinragenden Stutzen (17) definiert ist,
dadurch gekennzeichnet,
daß die zweite Gasauslaßöffnung (20) in bezug auf die Gaseinlaßöffnung (13) des zweiten Gasanalysators (10) seitlich angeordnet ist.

6. Einrichtung nach einem der Ansprüche 1 bis 5,
dadurch gekennzeichnet,
daß die zweite Gasauslaßöffnung (20) der Einrichtung (12) mit einer in der Gasauslaßöffnung (14) ausgebildeten Öffnung (19) des zweiten Gasanalysators (10) kommuniziert.

7. Einrichtung nach Anspruch 6,
dadurch gekennzeichnet,
daß die beiden Öffnungen (19, 20) annähernd koaxial zueinander angeordnet sind.

8. Einrichtung nach einem der Ansprüche 1 bis 7,
dadurch gekennzeichnet,
daß die Einrichtung (12) ein rotationssymmetrischer Körper ist, bei dem die Gaseinlaßöffnung (14) koaxial zur Achse (34) angeordnet ist, während der Einlaßstutzen (17) der Gaseinlaßöffnung (13) des zweiten Gasanalysators (10) parallel zur Rotationsachse (34) verläuft.

9. Einrichtung nach einem der Ansprüche 1 bis 8,
dadurch gekennzeichnet,
daß die zweite Gasauslaßöffnung (20) im zylindrischen Mantel des Hohlkörpers (21) ausgebildet ist, wobei ihre Achse in etwa rechtwinkelig zur Rotationsachse verläuft.

10. Gasanalysator mit einer Gaseinlaß- und einer Gasauslaßöffnung zur Untersuchung der Trübung von Rauchgasen, insbesondere zur Abgasuntersuchung von Dieselmotoren, mit einer Meßanordnung, durch die das zu untersuchende Gas geleitet wird, wobei das von der Meßanordnung ermittelte Untersuchungsergebnis in einer signalverarbeitenden Anordnung in Abhängigkeit von anderen Meßergebnissen korrigiert wird, gekennzeichnet durch eine an die Gaseinlaßöffnung angeschlossene Einrichtung nach einem der Ansprüche 1 bis 9.
